# EUROPEAN PATENT SPECIFICATION

(11) **EP 1 471 355 B1**
(45) Date of publication and mention of the grant of the patent: **28.02.2007**
(21) Application number: 03703128.3
(22) Date of filing: 31.01.2003
(51) Int. Cl.: G01N 33/53, G01N 37/00, C12N 15/00, C12Q 1/68

(54) **METHOD OF FIXING BIOMOLECULE TO CARRIER**
VERFAHREN ZUM ANHEFTEN EINES BIOMOLEKÜLS AN EINEN TRÄGER
PROCEDE DE FIXATION D'UNE BIOMOLECULE SUR UN SUPPORT

(30) Priority: 01.02.2002 JP 2002025622; 22.08.2002 JP 2002242456
(43) Date of publication of application: 27.10.2004
(73) Proprietor: Nisshinbo Industries, Inc., Chuo-ku, Tokyo 103-8650 (JP)
(72) Inventor: ODA, Ryuichi, NISSHINBO INDUSTRIES, INC., Chiba-shi, Chiba 267-0056 (JP); KIMURA, Naoki, NISSHINBO INDUSTRIES, INC., Chiba-shi, Chiba 267-0056 (JP)
(74) Representative: Tombling, Adrian George
(86) International application number: PCT/JP2003/001006
(87) International publication number: WO 2003/065040

(56) References cited:
- EP-A- 1 130 121
- WO-A-01/62963
- JP-A- 1 242 965
- JP-A- 10 104 230
- JP-A- 2001 083 163
- JP-A- 2001 136 968
- JP-A- 2001 281 246
- US-A- 5 932 711

### SEQUENCE LISTING

<110> Nisshinbo Industries, Inc.
<120> Method for immobilizing biological molecule on substrate
<130> F2037-1441
<140>
   <141> 2003-01-31
<150> JP 2002-25622
   <151> 2002-02-01
<150> JP 2002-242456
   <151> 2002-08-22
<160> 8
<170> Patent In version 3.0
<210> 1
   <211> 21
   <212> DNA
   <213> Artificial/Unknown
<220>
   <223> Description of Artificial Sequence: capture oligonucleotide
<400> 1
   aaatgggtac tgtgcctgtt a 21
<210> 2
   <211> 21
   <212> DNA
   <213> Artificial/Unknown
<220>
   <223> Description of Artificial Sequence : capture oligonucleotide
<400> 2
   atgactaccg gcgcgacgat g 21
<210> 3
   <211> 21
   <212> DNA
   <213> Artificial/Unknown
<220>
   <223> Description of Artificial Sequence: probe DNA
<400> 3 
tcgcccgctg tttttgatga ggcggatttt ccggcagttg ccgtttatct caccggcgct 60
gaatacacgg gcgaagagct ggacagcgat acctggcagg cggagctgca tatcgaagtt 120
ttcctgcctg ctcaggtgcc ggattcagag ctggatgcgt ggatggagtc ccggatttat 180
ccggtgatga gcgatatccc ggcactgtca gatttgatca ccagtatggt ggccagcggc 240
tatgactacc ggcgcgacga tg 262
<210> 4
   <211> 31
   <212> DNA
   <213> Artificial/Unknown
<220>
   <223> Description of Artificial Sequence: capture oligonucleotide
<400> 4
   tttttttttt aaatgggtac tgtgcctgtt a 31
<210> 5
   <211> 31
   <212> DNA
   <213> Artificial/Unknown
<220>
   <223> Description of Artificial Sequence: capture oligonucleotide
<400> 5
   tttttttttt atgactaccg gcgcgacgat g 31
<210> 6
   <211> 31
   <212> DNA
   <213> Artificial/Unknown
<220>
   <223> Description of Artificial Sequence: capture oligonucleotide
<400> 6
   tttttttttt atgactacca gcgcgacgat g 31
<210> 7
   <211> 21
   <212> DNA
   <213> Artificial/Unknown
<220>
   <223> Description of Artificial Sequence: capture oligonucleotide
<400> 7
   tcgccccgct gtttttgatg a 21
<210> 8
   <211> 21
   <212> DNA
   <213> Artificial/Unknown
<220>
   <223> Description of Artificial Sequence: capture oligonucleotide
<400> 8
   catcgtcgcg ccggtagtca t 21

## Description

### Technical Field

The present invention relates to a method for immobilizing a nucleic acid on a carrier. The method of the present invention is useful for operations of analysis of nucleic acids based on hybridization and so forth.

### Background Art

In analyses of nucleic acids based on hybridization, immunoassays and so forth, techniques of immobilizing nucleic acids or proteins on a carrier such as membranes and plates have conventionally been utilized. As such methods for immobilizing biomolecules, the following methods are known for nucleic acids:
(1) A method of chemically binding a nucleic acid introduced with a modification group, such as immobilization by a disulfide bond between a nucleic acid having a thiol group at the 5' end and a bead-like base material having thiol groups (P.J.R. Day, P.S. Flora, J.E. Fox, M.R. Walker, Biochem. J., 278, 735-740 (1991));
(2) A method of immobilizing a nucleic acid by adsorption on a carrier such as nitrocellulose, nylon membrane, or glass coated with a cation polymer such as poly-L-Lysine through ultraviolet (UV) irradiation or heat treatment (J. Sambrook, E.F. Fritsch and T. Maniatis, Molecular Cloning, Cold Spring Harbor Laboratory Press, Second Edition, pages 2.109-2.113 and pages 9.34-9.46, International Patent Publication in Japanese (Kohyo) No. 10-503841);
(3) A method of attaining immobilization on the basis of physical adsorption obtained by injecting a nucleic acid into wells of a microplate treated with a polylysine solution and heating the plate at 37°C (G.C.N. Parry and A.D.B. Malcolm, Biochem. Soc. Trans., 17, 230-231 (1989));
(4) A method of synthesizing DNA on a base material by using nucleotides which bonded to the base material (WO97/10365);
(5) A method of immobilizing a nucleic acid on a base material such as glass carrying a polymer compound having carbodiimide groups (Japanese Patent Laid-open No. 8-23975); and
(6) A method of immobilizing a nucleic acid onto a polymeric material using UV irradiation having a wavelength of 220 nm to 380 nm (European patent application EP-A-1130121) or 1 nm to 480 nm (WO 01/62963).

However, these methods suffer from drawbacks. That is, the method of (1) requires an extremely special apparatus and regents.

Furthermore, in the methods of (2) and (3), nucleic acids are dropped off from the carriers during the hybridization, in particular, in operation processes, and thus detection sensitivity may be reduced, or reproducibility cannot be obtained. Furthermore, these methods suffer from another drawback, that is, although a long nucleic acid can be immobilized, a short nucleic acid of about 50-mer or shorter such as oligomers cannot be efficiently immobilized. In these methods, the UV dose is about several tens mJ/cm². Further, the method of (4) also requires an extremely special apparatus and regents for synthesizing DNA on a base material, and the nucleic acid that can be synthesized is limited to about 25-mer or shorter. Moreover, in the method of (5), the material of the base material is limited, and a surface coating step is required.

Meanwhile, a technique of hydrophilizing a surface of a polystyrene film by irradiation of a vacuum ultraviolet ray on the surface has been reported (Hozumi et al., Hyomen Gijutsu (Surface Techniques)", vol. 52, No. 12, pp. 97-98, 2001). However, it has not been disclosed that a nucleic acid can be immobilized on a synthetic resin carrier by irradiation of an ultraviolet ray having a wavelength of about 280 nm.

### Disclosure of the Invention

In view of the aforementioned technical situations of the conventional techniques, an object of the present invention is to provide a method for conveniently and efficiently immobilizing a nucleic acid, in particular, a nucleic acid of a short chain length, on a carrier.

The inventors of the present invention conducted various researches in order to achieve the aforementioned object. As a result, they found that a nucleic acid can be efficiently immobilized on a carrier by spotting a solution of the nucleic acid on a carrier made of a synthetic resin and then irradiating the carrier with an ultraviolet ray, and accomplished the present invention.

Thus, the present invention provides the followings.
(1) A method for immobilizing a nucleic acid on a carrier, comprising the steps of spotting a solution of the nucleic acid on the carrier, and irradiating the carrier spotted with the solution of the nucleic acid with an ultraviolet ray containing a component having a wavelength of 280 nm, wherein the carrier is made of a synthetic resin.
(2) The method according to (1), wherein the synthetic resin is selected from thermoplastic resins, thermosetting resins, and copolymers.
(3) The method according to (2), wherein the synthetic resin is selected from polycarbonate, polymethyl methacrylate, acrylonitrile/butadiene/styrene copolymer, polyethylene, polyethylene terephthalate, polyphenol, polystyrene, polyacrylonitrile, polyvinyl chloride and aramide.
(4) The method according to any one of (1) to (3), wherein irradiation dose of the ultraviolet ray is 100 mJ/cm² or more.
(5) A method for producing a nucleic acid-immobilized carrier in which a nucleic acid is immobilized on a carrier, comprising the steps of spotting a solution of the nucleic acid on the carrier, and irradiating the carrier spotted with the solution of the nucleic acid with an ultraviolet ray containing a component having a wavelength of 280 nm to immobilize the nucleic acid on the carrier.
(6) The method according to (5), wherein the nucleic acid-immobilized carrier is used for analysis of the nucleic acid by hybridization.

Hereafter, the present invention will be explained in detail.

The carrier used for the present invention is for immobilizing a nucleic acid, and is characterized by being made of a synthetic resin. The synthetic resin is not particularly limited, so long as a synthetic resin enabling immobilization of a nucleic acid by ultraviolet ray irradiation is chosen. Specifically, examples include thermoplastic resins, thermosetting resins, copolymers and so forth.

More specifically, examples of the thermoplastic resins include ionomers (styrene type, olefin type), polynorbornenes, polyacetals, polyallylates, polyether ether ketones, polyethylene oxides, polyoxymethylenes, polyethylene terephthalates, polycarbonates, polystyrenes, polysulfones, polyparamethylstyrenes, polyallylamines, polyphenylene ethers, polyphenylene sulfides, polybutadienes, polybutylene terephthalates, polypropylenes, polymethylpentenes, polyether sulfones, polyphenylene sulfides, polyoxybenzoyls, polyoxyethylenes, cellulose acetates, polydimethylsiloxanes, polyisobutylenes, cellulose triacetates, poly-p-phenylene terephthalamides, polyisoprenes, polyacrylonitriles, polymethylpentenes, chlorinated plastics (polyvinyl chlorides, polyethylene chlorides, chlorinated polypropylenes, polyvinylidene chlorides), fluorinated plastics (polytetrafluoroethylenes, polychlorotrifluoroethylenes, polyvinylidene fluorides), polyamides (nylon 6, nylon 66), polyamideimides, polyimides (thermoplastic polyimides, polyether imides), polyethylene plastics (chlorinated, high density, low density), polyvinyl plastics (polyvinyl chloride, polyvinyl acetates, polyparavinylphenols, polyvinyl alcohols, polyvinyl ethers, polyvinylbutyrals, polyvinylformals), liquid crystal polymers (polyester type liquid crystal polymers), acrylate plastics (aminopolyacrylamides, polymethyl acrylates, polymethyl methacrylates, ethylpoly methacrylates, butylpolymethacrylates, thermoplastic elastomers (styrene type, olefin type, urethane type, polyester type, polyamide type, 1,2-polybutadiene type, vinyl chloride type, fluorinated type, polyionomer type, chlorinated polyethylene type, silicone type), and so forth.

Examples of the thermosetting resins include epoxy resins, polyxylenes, polyguanamines, polydiallyl phthalates, polyvinyl esters, polyphenols, unsaturated polyesters, polyfurans, polyimides, polyurethanes, poly maleates, melamine resins, urea resins, alkyd resins, benzoguanamine resins, polycyanates, polyisocyanates and so forth.

Examples of the copolymers include isobutylene/maleic anhydride copolymers, acrylonitrile/acrylate/styrene copolymers, acrylonitrile/EPDM/styrene copolymers, acrylonitrile/styrene copolymers, acrylonitrile/butadiene/styrene copolymers, butadiene/styrene/methyl methacrylate copolymers, ethylene/vinyl chloride copolymers, ethylene/vinyl acetate copolymers, ethylene/ethyl acrylate copolymers, acrylonitrile/butadiene/styrene copolymers, polyether ether ketone copolymers, fluorinated ethylene/polypropylene copolymers, tetrafluoroethylene/perfluoroalkyl vinyl ether copolymers, tetrafluoroethylene/ethylene copolymers, and so forth.

Among the aforementioned synthetic resins, particularly preferred are polycarbonates, polymethyl methacrylates, acrylonitrile/butadiene/styrene copolymers, polyethylenes, polyethylene terephthalates, polyphenols, polystyrenes, polyacrylonitriles, polyvinyl chlorides, aramides, and so forth.

Moreover, as the aforementioned synthetic resin, a synthetic resin added with a dye, coloring agent, plasticizer, pigment, polymerization inhibitor, surface-modifying agent, stabilizer, adhesion-imparting agent, heat-curing agent, dispersing agent, ultraviolet ray degradation-preventing agent, etc., as required, may be used. Furthermore, the aforementioned synthetic resin may consist of laminated two or more different kinds of the aforementioned synthetic resins in order to maintain the shape, or may consist of a single kind of synthetic resin. Moreover, the synthetic resin may consist of a polymer alloy obtained by mixing two or more kinds of the aforementioned synthetic resins. Furthermore, the aforementioned synthetic resin may be mixed with fibers such as venation fibers, fruit fibers, animal hair fibers, cocoon fibers, feather fibers, chitin fibers, chitosan fibers, and asbestos fibers.

The shape of the aforementioned carrier is not particularly limited, and examples of the shape include those of fiber, plate, filter, bead and so forth. Furthermore, it may be the shape of microtiter plate. In order to facilitate preservation of the obtained result, a back surface of plate or the like may be, for example, applied or coated with a material usable as a seal or the like (adhesives etc.) so that the material can be used as a seal.

A solution of a nucleic acid is spotted on predetermined positions of the aforementioned carrier.

The nucleic acid is not particularly different from a usual solid phase-immobilized nucleic acid used for hybridization of nucleic acids using a solid phase-immobilized nucleic acid, and it is not particularly limited so long as it is a nucleic acid that allows hybridization. For example, it may be a naturally occurring or synthesized DNA (including oligonucleotides) or RNA (including oligonucleotides). Further, it may be single-stranded or double-stranded. The chain length of the nucleic acid is not also particularly limited so long as it allows hybridization. However, it is usually about 5 to 50,000 nucleotides, preferably 20 to 10,000 nucleotides. Further, the nucleic acid may have a polymer of oligonucleotides having a group that becomes reactive upon ultraviolet ray irradiation such as those of thymidine or the like at the 5' end or 3' end.

The solvent for dissolving the nucleic acid is not also particularly limited, and examples include distilled water, buffers usually used for preparing a nucleic acid solution, for example, a Tris buffer such as TE buffer (10 mM Tris/hydrochloric acid, pH 8.0, 1 mM EDTA), a solution containing sodium chloride, a solution containing a carboxylic acid salt (sodium citrate, ammonium citrate, sodium acetate etc.), a solution containing a sulfonic acid salt (sodium dodecylsulfate, ammonium dodecylsulfate etc.), a solution containing a phosphonic acid salt (sodium phosphate, ammonium phosphate etc.) and so forth. Examples further include commercially available solvents such as Micro Spotting Solution (TeleCHem International, Inc.). Further, although the concentration of the nucleic acid solution is not particularly limited either, it is usually a concentration of 1 mmol/ml to 1 fmol/ml, preferably 100 pmol/ml to 100 fmol/ml.

Examples of the method for spotting of the nucleic acid solution on the carrier include a method of dropping the nucleic acid solution onto the carrier with a pipette, a method of using a commercially available spotter, and so forth. Although the shape of spot and amount of the solution to be spotted are not particularly limited so long as the position at which the nucleic acid solution has been spotted can be confirmed, the shape is preferably a dot shape or circular shape. The amount of the solution to be spotted is preferably 10 nl to 10 ml. The nucleic acid solution is spotted on one place or two or more places on the carrier. One kind of nucleic acid solution or two or more kinds of nucleic acid solutions may be spotted. A labeled nucleic acid may be immobilized as a positive control indicating immobilization of the nucleic acid on the carrier.

In a preferred embodiment of the present invention, after the nucleic acid solution is spotted on the carrier, an ultraviolet ray containing a component having a wavelength of 280 nm is irradiated. The nucleic acid solution can be dried after the spotting and before the ultraviolet ray irradiation. As for the method for drying the nucleic acid solution, it may be spontaneously dried, or dried by heating. When it is heated, the heating temperature is usually 30 to 100°C, preferably 35 to 45° C.

Then, an ultraviolet ray containing a component having a wavelength of 280 nm is irradiated at least on the position or positions of the carrier at which the nucleic acid has been immobilized. Specifically, the ultraviolet ray may be an ultraviolet ray having a broad waveform and containing a component having a wavelength of 280 nm. The irradiation dose is usually 100 mJ/cm² or more, preferably 200 mJ/cm² or more, as cumulative irradiation dose.

By immobilizing a nucleic acid on a carrier as described above, a nucleic acid-immobilized carrier is produced. The nucleic acid-immobilized carrier obtained by the method of the present invention can be used for, for example, analysis of nucleic acids based on hybridization. Because a nucleic acid immobilized on a carrier by the method of the present invention hardly detach from the carrier under the conditions of usual hybridization, more favorable detection sensitivity and reproducibility can be obtained compared with the case where the ultraviolet ray irradiation is not performed. The hybridization and detection thereof can be performed in the same manner as hybridization utilizing a usual solid phase-immobilized nucleic acid.

Since an inexpensive synthetic resin is used as a carrier for immobilizing a nucleic acid in the present invention, the cost can be reduced. Moreover, since a synthetic resin has a small density and can be easily formed, it becomes easy to produce DNA microarrays of various shapes. Further, it can be stored for a long period of time, and has superior storage stability. Furthermore, the method of the present invention does not require a step of coating a surface of carrier, and thus the procedure is simple.

### Brief Explanation of the Drawing

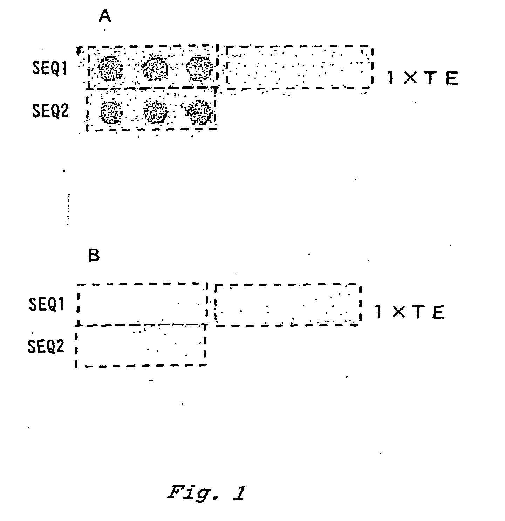

Fig. 1 (photograph) shows the result of hybridization using the oligonucleotide-immobilized plate produced in the example. A: Example 1, B: Comparative Example 1, SEQ1 : SEQ ID NO: 1, SEQ2: SEQ ID NO: 2. The dotted line represents the regions on which the oligonucleotides were immobilized, and the regions on which 1 x TE buffer solution was spotted as a control.

### Best Mode for Carrying out the Invention

Hereafter, the present invention will be explained more specifically by way of examples.

### Example 1: Immobilization of nucleic acid on plate

Oligonucleotides having the nucleotide sequences of SEQ ID NOS: 1 and 2 respectively (21-mers) were synthesized in a conventional manner by using an oligonucleotide synthesizer (Perkin-Elmer Applied Biosystems). Furthermore, DNA having the nucleotide sequence of SEQ ID NO: 3 (262 bp) was also prepared as a probe. The oligonucleotide having the nucleotide sequence of SEQ ID NO: 1 and the probe were biotinylated at the 5' ends. The oligonucleotide having the nucleotide sequence of SEQ ID NO: 2 was complementary to the biotinylated probe. These oligonucleotides were dissolved in 1 x TE buffer (10 mM Tris-HCl, pH 8, 1 mM EDTA) at a concentration of 1 pmol/µl.

Each of the aforementioned oligonucleotide solutions was spotted on a commercially available polymethyl methacrylate plate as three spots at predetermined positions (Fig. 1). The amount of the solutions used for each spotting was 0.5 µl, and the size of the spots was about 1 mm in diameter. This plate was put into a drier and dried at 42°C for 20 minutes. Then, an ultraviolet ray containing a component having a wavelength of 280 nm was irradiated for 200 mJ/cm² by using Uvstratalinker 2400 (STRATAGENE) at a distance of 16 cm. The irradiation time was 80 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 1

The polymethyl methacrylate plate was irradiated beforehand with an ultraviolet ray containing a component having a wavelength of 280 nm for 200 mJ/cm² by using Uvstratalinker 2400 (STRATAGENE) at a distance of 16 cm. Each of the oligonucleotide solutions described in Example 1 was spotted on the polymethyl methacrylate plate as three spots at predetermined positions (Fig. 1). The amount of the solutions used for each spotting was 0.5 µl, and the size of the spots was about 1 mm in diameter. The irradiation time was 80 seconds. This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 2: Hybridization and detection thereof

### (1) Hybridization

On the nucleic acid-immobilized portions of the oligonucleotide-immobilized plates of Example 1 and Comparative Example 1, 60 µl of a hybridization solution (Arrayit UniHyb, TeleCHem International, Inc.) containing 3 pmol of the biotinylated probe (262 bp) was placed, and the plates were put into a case shielded from water (HybriCassette), immersed in a water bath with the case, and heated at 45°C for 2 hours.

### (2) Post-hybridization

After the hybridization, post-hybridization washing was performed under the following conditions to remove the probe non-specifically adsorbed on the oligonucleotide-immobilized plates.

### [Post-hybridization washing conditions]

1) 2 x SSC, 0.1% SDS; room temperature, 5 minutes, 2 times
2) 0.2 x SSC, 0.1% SDS; 40°C, 5 minutes, 2 times
3) 2 x SSC; room temperature, 1 minute, 3 times

### (3) Detection of oligonucleotides immobilized on plate and hybridization

On the portions of the plates on which the hybridization solution was placed, 1.5 ml of a blocking solution containing milk proteins (BlockAce, Snow Brand Milk Products) was placed to perform blocking at room temperature for 30 minutes. After the blocking solution was removed, 1.5 ml of streptavidin-alkaline phosphatase conjugate solution (VECTOR) was placed and reacted at room temperature for 30 minutes. Then, the plates were immersed in TBST solution (50 mM Tris-HCl (pH 7.5), 0.15 M NaCl, 0.05% Tween 20) and shaken for 5 minutes to remove the conjugate that did not react. Finally, 1.5 ml of a substrate (TMB) solution was placed on the portions of the plates on which the hybridization solution was placed and left for 30 minutes to perform a coloring reaction.

The results are shown in Table 1 together with the results of Comparative Example 1. The symbols used in Table 1 have the same meanings also in Table 2 and the other tables mentioned later. The signals of the positions at which the oligonucleotide having the sequence of SEQ ID NO: 1 was immobilized indicate amounts of immobilized oligonucleotides, and the signals of the positions at which the oligonucleotide having the sequence of SEQ ID NO: 2 was immobilized indicate intensities of hybridization.

**Table 1**

| | Immobilized oligonucleotide | |
|---|---|---|
| | SEQ ID NO: 1 | SEQ ID NO: 2 |
| Example 1 | Ⓞ | Ⓞ |
| Comparative Example 1 | × | × |

| | | |
|---|---|---|
| Ⓞ: Most of signals appeared extremely clearly with extremely high sensitivity. ○: Most of signals appeared clearly with high sensitivity. Δ: A part of signals appeared unclearly or with low sensitivity. ×: Most of signals appeared unclearly or with low sensitivity, or no signal appeared at all. | | |

As evident from the results shown in Table 1, it can be seen that the oligonucleotides were more surely immobilized on the oligonucleotide-immobilized plate of Example 1 compared with the oligonucleotide-immobilized plate of Comparative Example 1. Moreover, on the oligonucleotide-immobilized plate of Example 1, the hybridization signals also appeared clearly. In addition, on the positions of control (positions at which a solution not containing any nucleic acid was spotted), no signal appeared at all.

### Example 3: Immobilization of nucleic acid on plate

Oligonucleotides having the nucleotide sequences of SEQ ID NOS: 4, 5 and 6 respectively (31-mers) were synthesized in a conventional manner by using an oligonucleotide synthesizer (Perkin-Elmer Applied Biosystems). The oligonucleotide having the nucleotide sequence of SEQ ID NO: 4 was biotinylated at the 5' end. The oligonucleotides having the nucleotide sequences of SEQ ID NOS: 4 and 5 corresponded to the oligonucleotides having the nucleotide sequences of SEQ ID NO: 1 and 2 described in Example 1 with ten thymidine residues at the 5' ends, respectively. The oligonucleotide of SEQ ID NO: 5 was complementary to the aforementioned biotinylated probe, and the oligonucleotide of SEQ ID NO: 6 did not have complementarity because it was different from the oligonucleotide of SEQ ID NO: 5 by one nucleotide. These oligonucleotides were dissolved in 3 x SSC at a concentration of 100 pmol/ml.

Each of the aforementioned oligonucleotide solutions was spotted on a commercially available polycarbonate plate as three spots at predetermined positions by using a spotter (Pyxsis 5500, CARTESIAN). The size of the spots was about 0.3 mm in diameter. This plate was put into a drier and dried at 42°C for 20 minutes. Then, an ultraviolet ray containing a component having a wavelength of 280 nm was irradiated for 300 mJ/cm² by using Uvstratalinker 2400 (STRATAGENE) at a distance of 16 cm. The irradiation time was 120 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 2

The polycarbonate plate was irradiated beforehand with an ultraviolet ray containing a component having a wavelength of 280 nm for 300 mJ/cm² by using Uvstratalinker 2400 (STRATAGENE) at a distance of 16 cm. Each of the oligonucleotide solutions described in Example 3 was spotted on the polycarbonate plate as three spots at predetermined positions by using a spotter (Pyxsis 5500, CARTESIAN). The irradiation time was 120 seconds. This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 4: Hybridization and detection thereof

On the nucleic acid-immobilized portions of the oligonucleotide-immobilized plates of Example 3 and Comparative Example 2, 60 ml of a hybridization solution (Arrayit UniHyb, TeleCHem International, Inc.) containing 3 pmol of the biotinylated probe (262 bp) was placed, and the plates were put into a case shielded from water (HybriCassette), immersed in a water bath with the case, and heated at 45°C for 2 hours.

Thereafter, post-hybridization, detection of the oligonucleotides immobilized on the plates and hybridization were performed in the same manner as that of Example 2. The results are shown in Table 2 together with the results of Comparative Example 2. The signals of the positions at which the oligonucleotide having the sequence of SEQ ID NO: 4 was immobilized indicate amounts of immobilized oligonucleotides, and the signals of the positions at which the oligonucleotide having the sequence of SEQ ID NO: 5 was immobilized indicate intensities of hybridization.

**Table 2**

| | Immobilized oligonucleotide | | |
|---|---|---|---|
| | SEQ ID NO: 4 | SEQ ID NO: 5 | SEQ ID NO: 6 |
| Example 3 | Ⓞ | Ⓞ | × |
| Comparative Example 2 | × | × | × |

As evident from the results shown in Table 2, it can be seen that the oligonucleotides were more surely immobilized on the oligonucleotide-immobilized plate of Example 3 compared with the oligonucleotide-immobilized plate of Comparative Example 2. Moreover, on the oligonucleotide-immobilized plate of Example 3, the hybridization signals also appeared clearly. In addition, on the positions of control (positions at which a solution not containing any nucleic acid was spotted) and SEQ ID NO: 6, no signal appeared at all.

### Example 5: Immobilization of nucleic acid on plate

Each of the aforementioned oligonucleotide solutions was spotted on a plate consisting of three layers of aramide non-woven fabric, Tetron cloth and aramide non-woven fabric (the surface consisted of aramide non-woven fabric, Sagami PCI) as three spots at predetermined positions by using a spotter (Pyxsis 5500, CARTESIAN). Tetron (registered trademark of Teijin, Ltd.) consists of polyethylene terephthalate. The size of the spots was about 0.3 mm in diameter. This plate was put into a drier and dried at 42°C for 20 minutes. Then, an ultraviolet ray containing a component having a wavelength of 280 nm was irradiated for 400 mJ/cm² by using Uvstratalinker 2400 (STRATAGENE) at a distance of 16 cm. The irradiation time was 160 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 3

The plate consisting of three layers of aramide non-woven fabric, Tetron cloth and aramide non-woven fabric (the surface consisted of aramide non-woven fabric, Sagami PCI) was irradiated beforehand with an ultraviolet ray containing a component having a wavelength of 280 nm for 400 mJ/cm² by using Uvstratalinker 2400 (STRATAGENE) at a distance of 16 cm. Each of the oligonucleotide solutions described in Example 3 was spotted on the aramide non-woven fabric/Tetron cloth/aramide non-woven fabric plate as three spots at predetermined positions by using a spotter (Pyxsis 5500, CARTESIAN). The irradiation time was 160 seconds. This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 6: Hybridization and detection thereof

On the nucleic acid-immobilized portions of the oligonucleotide-immobilized plates of Example 5 and Comparative Example 3, 60 ml of a hybridization solution (Arrayit UniHyb, TeleCHem International, Inc.) containing 3 pmol of the biotinylated probe (262 bp) was placed, and the plates were put into a case shielded from water (HybriCassette), immersed in a water bath with the case, and heated at 45°C for 2 hours.

Thereafter, post-hybridization, detection of the oligonucleotides immobilized on the plates and hybridization were performed in the same manner as that of Example 2. The results are shown in Table 3 together with the results of Comparative Example 3.

**Table 3**

| | Immobilized oligonucleotide | | |
|---|---|---|---|
| | SEQ ID NO: 4 | SEQ ID NO: 5 | SEQ ID NO: 6 |
| Example 5 | Ⓞ | Ⓞ | × |
| Comparative Example 3 | × | × | × |

As evident from the results shown in Table 3, it can be seen that the oligonucleotides were more surely immobilized on the oligonucleotide-immobilized plate of Example 5 compared with the oligonucleotide-immobilized plate of Comparative Example 3. Moreover, on the oligonucleotide-immobilized plate of Example 5, the hybridization signals also appeared clearly. In addition, on the positions of control (positions at which a solution not containing any nucleic acid was spotted) and SEQ ID NO: 6, no signal appeared at all.

### Example 6: Immobilization of nucleic acid on plate

The oligonucleotides having the nucleotide sequences of SEQ ID NOS: 1 and 2 described in Example 1 were dissolved in 5 x SSC aqueous solution at a concentration of 70 pmol/ml.

Each of the aforementioned oligonucleotide solutions was spotted on a commercially available polyethylene plate as three spots at predetermined positions by using a spotter (Pyxsis 5500, CARTESIAN). The size of the spots was about 0.2 mm in diameter. This plate was put into a drier and dried at 42°C for 20 minutes. Then, an ultraviolet ray containing a component having a wavelength of 280 nm was irradiated for 250 mJ/cm² by using Uvstratalinker 2400 (STRATAGENE) at a distance of 16 cm. The irradiation time was 100 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 4

The polyethylene plate was irradiated beforehand with an ultraviolet ray containing a component having a wavelength of 280 nm for 250 mJ/cm² by using Uvstratalinker 2400 (STRATAGENE) at a distance of 16 cm. Each of the oligonucleotide solutions described in Example 6 was spotted on the polyethylene plate as three spots at predetermined positions. The irradiation time was 100 seconds. This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 7: Hybridization and detection thereof

On the nucleic acid-immobilized portions of the oligonucleotide-immobilized plates of Example 6 and Comparative Example 4, 60 ml of a hybridization solution (Arrayit UniHyb, TeleCHem International, Inc.) containing 3 pmol of the biotinylated probe was placed, and the plates were put into a case shielded from water (HybriCassette), immersed in a water bath with the case, and heated at 45°C for 2 hours.

Thereafter, post-hybridization, detection of the oligonucleotides immobilized on the plates and hybridization were performed in the same manner as that of Example 2. The results are shown in Table 4 together with the results of Comparative Example 4. The signals of the positions at which the oligonucleotides having the sequence of SEQ ID NO: 1 were immobilized indicate amounts of immobilized oligonucleotides, and the signals of the positions at which the oligonucleotides having the sequence of SEQ ID NO: 2 were immobilized indicate intensities of hybridization.

**Table 4**

| | Immobilized oligonucleotide | |
|---|---|---|
| | SEQ ID NO: 1 | SEQ ID NO: 2 |
| Example 6 | Ⓞ | Ⓞ |
| Comparative Example 4 | × | × |

As evident from the results shown in Table 4, it can be seen that the oligonucleotides were more surely immobilized on the oligonucleotide-immobilized plate of Example 6 compared with the oligonucleotide-immobilized plate of Comparative Example 4. Moreover, on the oligonucleotide-immobilized plate of Example 6, the hybridization signals also appeared clearly. In addition, on the positions of control (positions at which a solution not containing any nucleic acid was spotted), no signal appeared at all.

### Example 8: Immobilization of nucleic acid on plate

Each of the oligonucleotide solutions described in Example 6 was spotted on a commercially available phenol resin plate as three spots at predetermined positions by using a spotter (Pyxsis 5500, CARTESIAN). The size of the spots was about 0.4 mm in diameter. This plate was put into a drier and dried at 42°C for 20 minutes. Then, an ultraviolet ray containing a component having a wavelength of 280 nm was irradiated for 600 mJ/cm² by using Uvstratalinker 2400 (STRATAGENE) at a distance of 16 cm. The irradiation time was 240 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 5

The phenol resin plate was irradiated beforehand with an ultraviolet ray containing a component having a wavelength of 280 nm for 600 mJ/cm² by using Uvstratalinker 2400 (STRATAGENE) at a distance of 16 cm. Each of the oligonucleotide solutions described in Example 6 was spotted on the polyethylene plate as three spots at predetermined positions by using a spotter (Pyxsis 5500, CARTESIAN). The irradiation time was 240 seconds. This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 9: Hybridization and detection thereof

On the nucleic acid-immobilized portions of the oligonucleotide-immobilized plates of Example 8 and Comparative Example 5, 60 ml of a hybridization solution (Arrayit UniHyb, TeleCHem International, Inc.) containing 5 pmol of the biotinylated probe (262 bp) was placed, and the plates were put into a case shielded from water (HybriCassette), immersed in a water bath with the case, and heated at 45°C for 2 hours.

Thereafter, post-hybridization, detection of the oligonucleotides immobilized on the plates and hybridization were performed in the same manner as that of Example 2. The results are shown in Table 5 together with the results of Comparative Example 5.

**Table 5**

| | Immobilized oligonucleotide | |
|---|---|---|
| | SEQ ID NO: 1 | SEQ ID NO: 2 |
| Example 8 | Ⓞ | Ⓞ |
| Comparative Example 5 | × | × |

As evident from the results shown in Table 5, it can be seen that the oligonucleotides were more surely immobilized on the oligonucleotide-immobilized plate of Example 8 compared with the oligonucleotide-immobilized plate of Comparative Example 5. Moreover, on the oligonucleotide-immobilized plate of Example 8, the hybridization signals also appeared clearly. In addition, on the positions of control (positions at which a solution not containing any nucleic acid was spotted), no signal appeared at all.

### Example 10: Immobilization of nucleic acid on plate

Each of the oligonucleotide solutions described in Example 6 was spotted on a commercially available polystyrene plate as three spots at predetermined positions by using a spotter (Pyxsis 5500, CARTESIAN). The size of the spots was about 0.4 mm in diameter. This plate was put into a drier and dried at 42°C for 20 minutes. Then, an ultraviolet ray containing a component having a wavelength of 280 nm was irradiated for 600 mJ/cm² by using Uvstratalinker 2400 (STRATAGENE) at a distance of 16 cm. The irradiation time was 240 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 6

The polystyrene plate was irradiated beforehand with an ultraviolet ray containing a component having a wavelength of 280 nm for 600 mJ/cm² by using Uvstratalinker 2400 (STRATAGENE) at a distance of 16 cm. Each of the oligonucleotide solutions described in Example 6 was spotted on the polyethyrene plate as three spots at predetermined positions by using a spotter (Pyxsis 5500, CARTESIAN). The irradiation time was 240 seconds. This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 11: Hybridization and detection thereof

On the nucleic acid-immobilized portions of the oligonucleotide-immobilized plates of Example 10 and Comparative Example 6, 60 ml of a hybridization solution (Arrayit UniHyb, TeleCHem International, Inc.) containing 3 pmol of the biotinylated probe (262 bp) was placed, and the plates were put into a case shielded from water (HybriCassette), immersed in a water bath with the case, and heated at 45°C for 2 hours.

Thereafter, post-hybridization, detection of the oligonucleotides immobilized on the plates and hybridization were performed in the same manner as that of Example 2. The results are shown in Table 6 together with the results of Comparative Example 6.

**Table 6**

| | Immobilized oligonucleotide | |
|---|---|---|
| | SEQ ID NO: 1 | SEQ ID NO: 2 |
| Example 10 | Ⓞ | Ⓞ |
| Comparative Example 6 | × | × |

As evident from the results shown in Table 6, it can be seen that the oligonucleotides were more surely immobilized on the oligonucleotide-immobilized plate of Example 10 compared with the oligonucleotide-immobilized plate of Comparative Example 6. Moreover, on the oligonucleotide-immobilized plate of Example 10, the hybridization signals also appeared clearly. In addition, on the positions of control (positions at which a solution not containing any nucleic acid was spotted), no signal appeared at all.

### Example 12: Immobilization of nucleic acid on microtiter plate

Each of the oligonucleotide solutions described in Example 6 was spotted on a 24-well polystyrene microtiter plate (NUNC) as three spots at predetermined positions. The amount of the solutions used for each spotting was 0.5 ml, and the size of the spots was about 1 mm in diameter. This plate was put into a drier and dried at 42°C for 20 minutes. Then, an ultraviolet ray containing a component having a wavelength of 280 nm was irradiated for 1200 mJ/cm² by using Uvstratalinker 2400 (STRATAGENE) at a distance of 16 cm. The irradiation time was 480 seconds. Then, water was added to the predetermined positions of the microtiter plate in a volume of 1 ml for each position, and the plate was shaken for 30 minutes for washing, and dried.

On the other hand, a solution not containing any nucleic acid (1 x TE buffer) was also subjected to the immobilization operation as a control by spotting it on the microtiter plate in a similar manner.

### Comparative Example 7

The 24-well polystyrene microtiter plate (NUNC) was irradiated beforehand with an ultraviolet ray containing a component having a wavelength of 280 nm for 1200 mJ/cm² by using Uvstratalinker 2400 (STRATAGENE) at a distance of 16 cm. Each of the oligonucleotide solutions described in Example 6 was spotted on the polystyrene plate as three spots at predetermined positions. The amount of the solutions used for each spotting was 0.5 ml, and the size of the spots was about 1 mm in diameter. The irradiation time was 480 seconds. This plate was put into a drier and dried at 42°C for 20 minutes. Then, water was added to the predetermined positions of the microtiter plate in a volume of 1 ml for each position, and the plate was shaken for 30 minutes for washing, and dried.

On the other hand, a solution not containing any nucleic acid (1 x TE buffer) was also subjected to the immobilization operation as a control by spotting it on the plate in a similar manner.

### Example 13: Hybridization and detection thereof

On the nucleic acid-immobilized portions of the oligonucleotide-immobilized microtiter plates of Example 12 and Comparative Example 7, 100 ml of a hybridization solution (Arrayit UniHyb, TeleCHem International, Inc.) containing 3 pmol of the biotinylated probe (262 bp) was placed, a lid was placed on the plate so that the solution should be shielded from water, and the plate was immersed in a water bath and heated at 45°C for 2 hours.

Thereafter, post-hybridization, detection of the oligonucleotides immobilized on the microtiter plates and hybridization were performed in the same manner as that of Example 2. The results are shown in Table 7 together with the results of Comparative Example 7.

**Table 7**

| | Immobilized oligonucleotide | |
|---|---|---|
| | SEQ ID NO: 1 | SEQ ID NO: 2 |
| Example 12 | Ⓞ | Ⓞ |
| Comparative Example 7 | × | × |

As evident from the results shown in Table 7, it can be seen that the oligonucleotides were more surely immobilized on the oligonucleotide-immobilized microtiter plate of Example 12 compared with the oligonucleotide-immobilized microtiter plate of Comparative Example 7. Moreover, on the oligonucleotide-immobilized microtiter plate of Example 12, the hybridization signals also appeared clearly. In addition, on the positions of control (positions at which a solution not containing any nucleic acid was spotted), no signal appeared at all.

### Example 14: Immobilization of nucleic acid on polystyrene beads

Polystyrene beads (Dainippon Ink & Chemicals, Mitsubishi Chemical, etc.) were put into a quartz cell, and each of the oligonucleotide solutions described in Example 6 was introduced into the cell in a volume of 500 µl. Then, an ultraviolet ray containing a component having a wavelength of 280 nm was irradiated for 1200 mJ/cm² by using Uvstratalinker 2400 (STRATAGENE) at a distance of 16 cm. The irradiation time was 480 seconds. Then, water was added to the beads and shaken for 30 minutes to wash the beads, and the beads were dried.

On the other hand, a solution not containing any nucleic acid (1 x TE buffer) was also subjected to the immobilization operation as a control in a similar manner.

### Comparative Example 8

The polystyrene beads (Dainippon Ink & Chemicals, Mitsubishi Chemical, etc.) were put into a quartz cell, and an ultraviolet ray containing a component having a wavelength of 280 nm was irradiated beforehand for 1200 mJ/cm² by using Uvstratalinker 2400 (STRATAGENE) at a distance of 16 cm. The irradiation time was 480 seconds. Then, each of the oligonucleotide solutions described in Example 6 was introduced into the cell in a volume of 500 µl. The beads were taken out from the cell, then put into a drier and dried at 42°C for 20 minutes. Thereafter, water was added to the beads and shaken for 30 minutes to wash the beads, and the beads were dried.

On the other hand, a solution not containing any nucleic acid (1 x TE buffer) was also subjected to the immobilization operation as a control in a similar manner.

### Example 15: Hybridization and detection thereof

To the oligonucleotide-immobilized beads of Example 14 and Comparative Example 8, 100 ml of a hybridization solution (Arrayit UniHyb, TeleCHem International, Inc.) containing 3 pmol of the biotinylated probe (262 bp) was added, a lid was placed so that the beads and the solution should be shielded from water, and they were immersed in a water bath and heated at 45°C for 2 hours.

Thereafter, post-hybridization, detection of the oligonucleotides immobilized on the beads and hybridization were performed in the same manner as that of Example 2. The results are shown in Table 8 together with the results of Comparative Example 8. The signals of the beads on which the oligonucleotide having the sequence of SEQ ID NO: 1 was immobilized indicate amounts of immobilized oligonucleotides, and the signals of the beads on which the oligonucleotide having the sequence of SEQ ID NO: 2 was immobilized indicate intensities of hybridization.

**Table 8**

| | Immobilized oligonucleotide | |
|---|---|---|
| | SEQ ID NO: 1 | SEQ ID NO: 2 |
| Example 14 | Ⓞ | Ⓞ |
| Comparative Example 8 | × | × |

As evident from the results shown in Table 8, it can be seen that the oligonucleotides were more surely immobilized on the oligonucleotide-immobilized beads of Example 14 compared with the oligonucleotide-immobilized beads of Comparative Example 8. Moreover, on the oligonucleotide-immobilized beads of Example 14, the hybridization signals also appeared clearly. In addition, on the beads of control (beads immersed in a solution not containing any nucleic acid), no signal appeared at all.

### Example 16: Immobilization of nucleic acid on plate

A λDNA fragment (A) was amplified in a conventional manner by using oligonucleotides having the nucleotide sequences shown in SEQ ID NOS : 7 and 8 as primers. The obtained fragment was subjected to agarose electrophoresis and detected by ethidium bromide staining. As a result, it was found that the fragment had a length of about 300 b. Further, a λDNA fragment that was not complementary to the aforementioned λDNA, λDNA fragment (B) (about 300 b), was also amplified in a similar manner.

A solution of each of the aforementioned λDNAs was spotted on a commercially available polycarbonate plate as three spots at predetermined positions by using a spotter (Pyxsis 5500, CARTESIAN). The size of the spots was about 0.3 mm in diameter. This plate was put into a drier and dried at 42°C for 20 minutes. Then, an ultraviolet ray containing a component having a wavelength of 280 nm was irradiated for 600 mJ/cm² by using Uvstratalinker 2400 (STRATAGENE) at a distance of 16 cm. The irradiation time was 240 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 9

Each of the λDNA solutions described in Example 16 (concentration: 1 pmol/µl) was spotted on the polycarbonate plate as three spots at predetermined positions by using a spotter (Pyxsis 5500, CARTESIAN). This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 17: Hybridization and detection thereof

### (1) Hybridization

A λDNA fragment (C) was amplified by using an oligonucleotide having the nucleotide sequence shown in SEQ ID NO: 7 labeled with biotin at the 5' end and an oligonucleotide having the nucleotide sequence shown in SEQ ID NO: 8 as primers. The sequence of this λDNA fragment (C) was the same as that of the sequence of λDNA fragment (A) prepared in Example 16.

The λDNA-immobilized plates of Example 16 and Comparative Example 9 were immersed in water heated to 95°C for 5 minutes, and immersed in water cooled to 4°C for 5 minutes. Subsequently, on the nucleic acid-immobilized portions of the λDNA-immobilized plates, 60. ml of a hybridization solution (Arrayit UniHyb, TeleCHem International, Inc.) containing 1 pmol of the aforementioned biotinylated λDNA fragment (C) was placed, and the plates were put into a case shielded from water (HybriCassette), immersed in a water bath with the case, and heated at 60°C for 2 hours.

### (2) Post-hybridization

After the hybridization, post-hybridization washing was performed under the following conditions to remove the probe non-specifically adsorbed on the λDNA-immobilized plates.

### [Post-hybridization washing conditions]

1) 2 x SSC, 0.1% SDS; room temperature, 5 minutes, 2 times
2) 0.2 x SSC, 0.1% SDS; 40°C, 5 minutes, 2 times
3) 2 x SSC; room temperature, 1 minute, 3 times

### (3) Detection of hybridization

On the portions of the plates on which the hybridization solution was placed, 1.5 ml of a blocking solution containing milk proteins (BlockAce, Snow Brand Milk Products) was placed to perform blocking at room temperature for 30 minutes. After the blocking solution was removed, 1.5 ml of streptavidin-alkaline phosphatase conjugate solution (VECTOR) was placed and reacted at room temperature for 30 minutes. Then, the plates were immersed in TBST solution (50 mM Tris-HCl (pH 7.5), 0.15 M NaCl, 0.05% Tween 20) and shaken for 5 minutes to remove the conjugate that did not react. Finally, 1.5 ml of a substrate (TMB) solution was placed on the portions of the plates on which the hybridization solution was placed and left for 30 minutes to perform a coloring reaction.

The results are shown in Table 9.

**Table 9**

| | Immobilized nucleic acid | |
|---|---|---|
| | λDNA fragment (A) | λDNA fragment (B) |
| Example 16 | Ⓞ | × |
| Comparative Example 9 | × | × |

As evident from the results shown in Table 9, it can be seen that the λDNA fragments were surely immobilized on the λDNA-immobilized plate of Example 16, because the hybridization signals specifically and clearly appeared on the λDNA-immobilized plate of Example 16. On the other hand, on the λDNA-immobilized plate of Comparative Example 9, no signal appeared at all. In addition, on the positions of control (positions at which a solution not containing any nucleic acid was spotted) of the λDNA-immobilized plate of Example 16' and the λDNA-immobilized plate of Comparative Example 9, no signal appeared at all.

### Example 18: Immobilization of nucleic acid on plate

Each of the λDNA solutions described in Example 16 was spotted on a commercially available polymethyl methacrylate plate as three spots at predetermined positions by using a spotter (Pyxsis 5500, CARTESIAN). The size of the spots was about 0.3 mm in diameter. This plate was put into a drier and dried at 42°C for 20 minutes. Then, an ultraviolet ray containing a component having a wavelength of 280 nm was irradiated for 1200 mJ/cm² by using Uvstratalinker 2400 (STRATAGENE) at a distance of 16 cm. The irradiation time was 480 seconds. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Comparative Example 10

Each of the λDNA solutions described in Example 16 was spotted on the methacrylate plate as three spots at predetermined positions by using a spotter (Pyxsis 5500, CARTESIAN). This plate was put into a drier and dried at 42°C for 20 minutes. Then, the plate was washed by shaking in water for 30 minutes, and dried.

On the other hand, a solution not containing any nucleic acid (1 x TE buffer) was also subjected to the immobilization operation as a control by spotting it on the plate in a similar manner.

### Example 19: Hybridization and detection thereof

The λDNA-immobilized plates of Example 18 and Comparative Example 10 were immersed in water heated to 95°C for 10 minutes, and immersed in water cooled to 4°C for 5 minutes. Subsequently, on the nucleic acid-immobilized portions of the λDNA-immobilized plates, 60 ml of a hybridization solution (Arrayit UniHyb, TeleCHem International, Inc.) containing 1 pmol of the biotinylated λDNA (C) described in Example 17 was placed, and the plates were put into a case shielded from water (HybriCassette), immersed in a water bath with the case, and heated at 60°C for 2 hours.

Thereafter, post-hybridization and detection of hybridization were performed in the same manner as that of Example 17. The results are shown in Table 10.

**Table 10**

| | Immobilized nucleic acid | |
|---|---|---|
| | λDNA fragment (A) | λDNA fragment (B) |
| Example 18 | Ⓞ | × |
| Comparative Example 10 | × | × |

As evident from the results shown in Table 10, it can be seen that the λDNA fragments were surely immobilized on the λDNA-immobilized plate of Example 18, because the hybridization signals specifically and clearly appeared on the plate. On the other hand, no signal appeared at all on the λDNA-immobilized plate of Comparative Example 10. In addition, also on the positions of control (positions at which a solution not containing any nucleic acid was spotted) of the λDNA-immobilized plate of Example 18 and the λDNA-immobilized plate of Comparative Example 10, no signal appeared at all.

### Industrial Applicability

According to the method of the present invention, a nucleic acid, especially a short chain length nucleic acid, can be conveniently and efficiently immobilized on a synthetic resin carrier.

## Claims

1. A method for immobilizing a nucleic acid on a carrier, comprising the steps of spotting a solution of the nucleic acid on the carrier, and irradiating the carrier spotted with the solution of the nucleic acid with an ultraviolet ray containing a component having a wavelength of 280 nm, wherein the carrier is made of a synthetic resin or a natural resin.

2. The method according to claim 1, wherein the synthetic resin is selected from thermoplastic resins, thermosetting resins, and copolymers.

3. The method according to claim 2, wherein the synthetic resin is selected from polycarbonate, polymethyl methacrylate, acrylonitrile/butadiene/styrene copolymer, polyethylene, polyethylene terephthalate, polyphenol, polystyrene, polyacrylonitrile, polyvinyl chloride and aramide.

4. The method according to any one of claims 1 to 3, wherein irradiation dose of the ultraviolet ray is 100 mJ/cm² or more.

5. A method for producing a nucleic acid -immobilized carrier in which a nucleic acid is immobilized on a carrier, comprising the steps of spotting a solution of the nucleic acid on the carrier, and irradiating the carrier spotted with the solution of the nucleic acid with an ultraviolet ray containing a component having a wavelength of 280 nm to immobilize the nucleic acid on the carrier.

6. The method according to claim 5, wherein the nucleic acid-immobilized carrier is used for analysis of the nucleic acid by hybridization.

## Patentansprüche

1. Verfahren zum Immobilisieren einer Nukleinsäure auf einem Träger, umfassend die Schritte des Sprenkelns einer Lösung der Nukleinsäure auf den Träger und des Bestrahlens des Trägers, der mit der Lösung der Nukleinsäure besprenkelt ist, mit einem Ultraviolettstrahl, der eine Komponente aufweist, die eine Wellenlänge von 280 nm aufweist, wobei der Träger aus einem Kunstharz oder einem Naturharz hergestellt ist.

2. Verfahren nach Anspruch 1, wobei das Kunstharz aus thermoplastischen Harzen, aus in Wärme aushärtenden Harzen und aus Copolymeren ausgewählt ist.

3. Verfahren nach Anspruch 2, wobei das Kunstharz aus Polykarbonat, Polymethylmethacrylat, Acrylonitril/Butadien/Styrol-Copolymer, Polyethylen, Polyethylenterephthalat, Polyphenol, Polystyrol, Polyacrylonitril, Polyvinylchlorid und Aramid ausgewählt ist.

4. Verfahren nach einem der Ansprüche 1 bis 3, wobei die Bestrahlungsdosis des Ultraviolettstrahls 100 mJ/cm² oder mehr ist.

5. Verfahren zur Herstellung eines Trägers mit immobilisierter Nukleinsäure, bei dem eine Nukleinsäure auf einem Träger immobilisiert wird, umfassend die Schritte des Sprenkelns einer Lösung der Nukleinsäure auf den Träger und des Bestrahlens des Trägers, der mit der Lösung der Nukleinsäure besprenkelt ist, mit einem Ultraviolettstrahl, der eine Komponente aufweist, die eine Wellenlänge von 280 nm aufweist, um die Nukleinsäure auf dem Träger zu immobilisieren.

6. Verfahren nach Anspruch 5, wobei der Träger mit immobilisierter Nukleinsäure zur Analyse der Nukleinsäure mittels Hybridisierung verwendet wird.

## Revendications

1. Procédé d'immobilisation d'un acide nucléique sur un support, comprenant les étapes de formation de taches avec une solution de l'acide nucléique sur le support, et irradiation du support comportant les taches de solution de l'acide nucléique avec un rayonnement ultraviolet comprenant une composante ayant une longueur d'onde de 280 nm, dans lequel le support est réalisé en résine synthétique ou en résine naturelle.

2. Procédé selon la revendication 1, dans lequel la résine synthétique est choisie parmi les résines thermoplastiques, les résines thermodurcissables, et les copolymères.

3. Procédé selon la revendication 2, dans lequel la résine synthétique est choisie parmi le polycarbonate, le méthacrylate de polyméthyle, le copolymère d'acrylonitrile/butadiène/styrène, le polyéthylène, le polyéthylène téréphtalate, le polyphénol, le polystyrène, le polyacrylonitrile, le chlorure de polyvinyle et l'aramide.

4. Procédé selon l'une quelconque des revendications 1 à 3, dans lequel la dose d'irradiation du rayonnement ultraviolet est de 100 mJ/cm² ou plus.

5. Procédé de préparation d'un support sur lequel est immobilisé un acide nucléique, dans lequel un acide nucléique est immobilisé sur un support, présentant les étapes de formation de taches avec une solution de l'acide nucléique sur le support, et irradiation du support comportant les taches de solution de l'acide nucléique avec un rayonnement ultraviolet comprenant une composante ayant une longueur d'onde de 280 nm pour immobiliser l'acide nucléique sur le support.

6. Procédé selon la revendication 5, dans lequel le support sur lequel est immobilisé un acide nucléique est utilisé pour l'analyse de l'acide nucléique par hybridation.
